# EUROPEAN PATENT APPLICATION

(11) **EP 1 048 223 A1**
(43) Date of publication of application: **02.11.2000**
(21) Application number: 98967081.5
(22) Date of filing: 08.12.1998
(51) Int. Cl.: A23L 1/236

(54) **SWEETENER COMPOSITIONS CONTAINING ASPARTAME AND ASPARTAME DERIVATIVE**

(30) Priority: 15.12.1997 JP 34477897
(71) Applicant: Ajinomoto Co., Inc., Tokyo 104-0031 (JP)
(72) Inventor: KISHISHITA, Akihiro, Kanagawa-ken 210-0801 (JP); NAGASHIMA, Kazutaka, Kanagawa-ken 210-0801 (JP)
(74) Representative: Nash, David Allan
(86) International application number: PCT/JP98/05555
(87) International publication number: WO 99/30575

(57) **Abstract**

Excellent sweetener compositions containing aspartame (APM) and APM derivative and being similar to sucrose in tasting properties, which can be easily, uniformly and efficiently produced at a high purity by the process for producing N-[N-(3,3-dimethylbutyl)-L-α-aspartyl]-L-phenylalanine methyl ester by the reductive alkylation of APM and 3,3-dimethylbutyraldehyde which comprises using 3,3-dimethylbutyraldehyde in an amount at most equimolar to APM for producing N-[N-(3,3-dimethylbutyl)-L-α-aspartyl]-L-phenylalanine methyl ester from a portion of the APM existing in the reaction system, then separating the reducing agent from the liquid reaction mixture, and effecting crystallization followed by the separation of the crystals thus precipitated.

## Description

### TECHNICAL FIELD OF INVENTION

The present invention relates to a process for production of a sweetener composition containing the sweetening substances of aspartame (APM) and N-[N-(3,3-dimethylbutyl)-L-α-aspartyl]-L-phenylalanine methyl ester, which hereinafter may be abbreviated to "N-(3,3-dimethylbutyl)-APM", and simply referred to as "APM derivative", and the sweetener composition.

### BACKGROUND OF ART

In recent years, as eating habits have been improved to a high level, fatness caused by excessive sugar intake and diseases accompanied by fatness have been at issue. Accordingly, the development of a low-calory sweetening agent (sweetener) that replaces sugar has been in demand. The aspartame (APM) which is excellent in safety and quality of sweetness, is widely used as a sweetening agent. However, this is somewhat problematic in stability.

Under these circumstances, in the French Patent No.2697844 specification, it is stated that derivatives in which an alkyl group is introduced on an amino group of aspartic acid constituting the APM are studied in one approach to improve slightly the stability and to improve the sweetening potency, and among them N-(3,3-dimethylbutyl-APM is markedly improved in the sweetening potency. For the production of N-(3,3-dimethylbutyl)-APM, a process for alkylating APM reductively under the coexistence of 3,3-dimethylbutylaldehyde with sodium cyanoborohydride in methanol (refer to FR 2697844 specification), and a process for alkylating APM reductively under the coexistence of 3,3-dimethylbutylaldehyde with platinum carbon as the catalyst in a mixed solvent of water and methanol at a pH value in a range of 4.5 to 5 (refer to WO95/30689 specification) are known. And however, when a reaction is carried out according to the processes described in such above patent specifications, 3,3-dimethylbutylaldehyde and APM both unreacted, or N-[N,N-di(3,3-dimethylbutyl)-L-α-aspartyl]-L-phenylalanine methyl ester, wherein 2 alkyl groups have been introduced thereto are mixed in the reaction solution (mixture) or the crude crystals of the product therefrom, to no small extent.

The sweetening potencies of N-(3,3-dimethylbutyl)-APM being a sweetener having sweetness in a high degree and APM being an amino acid based sweetener, are reported to be 10,000 times by weight ratio (refer to Japanese Patent Kohyou Publication JP-A-8-503206) and about 200 times by weight ratio (refer to Japanese Patent Kokoku Publication JP-B-47-31031) that of sucrose, respectively.

The properties in quality of sweetness for N-(3,3-dimethylbutyl)-APM are not reported in details, and however such compound is extremely weak in early taste ( which means that a sweetener when put in the mouth tastes sweet as early as sucrose), and extremely strong in later taste (which means that a sweetener tastes sweet later than sucrose ), according to the present inventors' findings. It is strong in astringency (astringent taste) and badly-balanced for quality of sweetness as compared to sucrose. On the other hand, with respect to APM, its properties for quality of sweetness is weak in early taste, and strong in later taste, although they are not so bad as those in N-(3,3-dimethylbuty)-APM. Therefore, any one of both compounds has the properties for quality of sweetness which is weak in early taste, and strong in later taste, and thus is out of quality of sweetness, taking into account the standard that sucrose is natural in sweetness.

With respect to improvement in the properties for quality of sweetness, there are various proposals for the improvement mainly in later taste (For example, refer to Japanese Patent Kokai Publication JP-A-56-148255, JP-A-58-141760, JP-A-58-220668, etc.). Methods for obtaining a natural quality of sweetness closer to that in sucrose, for example, by the combination with sucrose (refer to Japanese Patent Kokai Publication JP-A-57-152862), etc. are found among the proposals. On the other hand, it is possible that early taste is intensified, later taste is weakened, and also astringent taste is weakened to take a balance in quality of sweetness by combining homogeneously and properly N-(3,3-dimethylbutyl)-APM being a sweetener having sweetness in a high degree with APM being an amino acid based sweetener, according to the findings found by the inventors in the present invention. Namely, by combining homogeneously and properly N-(3,3-dimethylbutyl)-APM which is weak in early taste with APM, it is improved in early taste and thereby is expected to give a sweetener having sweetness in a high degree and a quality of sweetness totally good in balance, closer to that of sucrose, as compared to N-(3,3-dimethylbutyl)-APM or APM each alone.

The sweetening potency of N-(3,3-dimethylbutyl)-APM is 10,000 times by weight ratio (refer to Japanese Patent Kohyou Publication JP-A-8-503206) as much as that of sucrose, and therefore it is problematic in adjusting a degree of sweetness while using it. Accordingly, a combination of N-(3,3-dimethylbutyl)-APM with APM which is proper for sweeteners is required in view of not only improvement in quality of sweetness, but also uses thereof.

### PROBLEM TO BE SOLVED BY INVENTION

In view of the properties in a quality of sweetness present in APM and APM derivative (N-(3,3-dimethylbutyl)-APM) described in the previous section, a problem to be solved by the present invention is to provide a process for producing conventionally and homogeneously (uniformly) a sweetener composition containing the APM and the N-(3,3-dimethylbutyl)-APM at a high yield and at a high purity which is improved in a quality of sweetness, as compared to the process for mixing crystals of APM with crystals of APM derivative, after each separately produced, and also to provide such an excellent sweetener composition thus obtained.

### DISCLOSURE OF INVENTION

In order to solve the problem in the present invention, the present inventors have studied earnestly and found the fact that in the process for producing N-[N-(3,3-dimethylbutyl)-L-α-aspartyl]-L-phenylalanine methyl ester by the reaction of APM with 3,3-dimethylbutylaldehyde for reductive alkylation, by using the 3,3-dimethylbutylaldehyde in an amount at most equimolar, preferably about 0.0003 - 0.28 mol, more preferably about 0.0005 - 0.25 mol to 1 mol of the APM in said reaction for producing the APM derivative (N-(3,3-dimethylbutyl)-APM) from a portion of the APM existing in the reaction system, then separating the reducing agent from the liquid reaction mixture, and effecting crystallization followed by the separation of the crystals thus precipitated, an excellent sweetener composition containing aspartame (APM) and N-[N-(3,3-dimethylbutyl)-L-α-aspartyl]-L-phenylalanine methyl ester and having preferable balance in a quality of sweetness, can be easily, uniformly and efficiently produced at a high yield and at a high purity, without producing N-[N,N-di(3,3-dimethylbutyl)-L-α-aspartyl]-L-phenylalanine methyl ester as a by-product, in which two alkyl groups are introduced, as compared to those in the process for producing crystals of APM and crystals of APM derivative, each separately, and thereafter mixing the both two types of crystals thus obtained separately, and thereby completed the present invention.

That is to say, the present invention is directed to in a process for production of N-[N-(3,3-dimethylbutyl)-L-α-aspartyl]-L-phenylalanine methyl ester comprising an reaction of APM with 3,3-dimethylbutylaldehyde for reductive alkylation, the process for production of a sweetener composition containing APM and said APM derivative, wherein said APM derivative is produced from a portion of APM by using said 3,3-dimethylbutylaldehyde in an amount at most equimolar to APM in said reaction, the reducing agent is separated from the reaction solution, and said solution is subjected to crystallization followed by the separation of the crystals thus precipitated.

According to the process for production thereof, a sweetener composition containing homogeneously APM and APM derivative can be produced.

In the present invention, the following contents are further contained:
1. A sweetener composition containing APM and APM derivative obtained in the above-mentioned process for production thereof in the present invention may be preferable for a sweetener.
2. The composition as produced mentioned above in the present invention, and is in the dried form, may be preferable in particular for a sweetener.
3. The above-mentioned sweetener composition obtained in the present invention, containing a carrier for sweeteners, if required, can be used for a sweetener or a sweetening agent. The sweetness can be given to a material requesting a sweetness by using the composition for a material requesting a sweetness for animals such as humans, for example, foods, cosmetics in the mouth (dental rinse, mouthwash, etc.), an oral pharmaceuticals, etc.
   In the production or selection of the carrier, if employed, a suitable carrier can be produced or selected through the known methods for production or selection of carriers therefor.
4. The above-mentioned sweetener composition, wherein said APM and said APM derivative are contained in a ratio of APM to APM derivative being 100 to about 0.05 - 50 by weight, may be preferable, particularly for use in a sweetener.
5. When the above-mentioned sweetener composition may be produced according to the above-mentioned process for production thereof, wherein said 3,3-dimethylbutylaldehyde is used in an amount of about 0.0003 - 0.28 mole, more preferably about 0.0005 - 0.25 per 1 mole of APM in said reaction for reductive alkylation, a particularly desirable sweetener composition can be obtained.

### EMBODIMENTS OF INVENTION

When a APM derivative (N-(3,3-dimethylbutyl)-APM) is produced in an reaction of APM with 3,3-dimethylbutylaldehyde for reductive alkylation, by using said 3,3-dimethylbutylaldehyde in an amount at most equimolar to APM, preferably in an amount of about 0.0003 - 0.28 mol, more preferably about 0.0005 - 0.25 mol per 1 mole of APM in said reaction, a portion of the APM present can be converted into the APM derivative efficiently without producing N-[N,N-di(3,3-dimethylbutyl)-L-α-aspartyl]-L-phenylalanine methyl ester as a by-product.

After the reaction is finished, when the catalyst used is separated off a solution containing APM and the APM derivative is obtained. By concentrating, if required, and subjecting the solution to crystallization followed by the separation of the crystals thus precipitated, a sweetener composition containing APM and the APM derivative, particularly containing homogeneously (uniformly) APM and the APM derivative, preferable for a sweetener can be produced.

For the catalyst employed in the reaction, platinum-carbon, palladium-carbon, platinum black, palladium black, etc. (refer to WO95/30689 specification) are known, and however, the species of the catalyst are not limited thereto. The catalyst which will be developed and provided in the future, can be also employed.

For the solvent usable in the reaction for the alkylation, one kind of solvent selected from the water, ethyl acetate, methyl acetate, acetic acid, toluene, hexane, tetrahydrofuran, acetnitrile, dimethoxyethane, ethyl ether, isopropyl alcohol, ethyl alcohol, methyl alcohol, dichloromethane, chloroform and 1,2-dichloroethane, or a mixed solvent obtained from 2 or more kinds of solvents selected from above these solvents.

It is necessary to make the concentration of APM in the reaction solution for crystallization higher, in order to make a yield therefor higher up. Therefore, it is desirable to employ an alcohol based solvent, or a mixed solvent obtained from the solvent and the other solvent(s) for the solvent used in the reaction. However, in case that there is a material or serious question as to the solvent remaining in the end product in view of quality of the product, water or the mixed solvent of water and alcohol based solvent(s) may be preferably employed.

For the after treatment of the reaction solution, first, the catalyst used in the reaction is separated from the solution. For the thus obtained reaction solution, when it may be obtained by the reaction using an alcohol based solvent or a mixed solvent of this solvent with the other solvent, such solvent is replaced by water as much as possible in order to minimize the solvent which may exist in the end product. This replacement is achieved by adding a suitable amount of water to the reaction solution, and then concentrating the same.

In the production of the sweetener composition, to control or regulate a weight ratio of the APM derivative per total amount of the APM and APM derivative contained in the end product of the composition, it is achieved by adjusting an amount of 3,3-dimethybbutylaldehyde, a reaction temperature and reaction time, or by adding before the starting of the reaction APM in a suitable and much amount in excess of the limit of its solubility, and dissolving the undissolved APM by heating or the like before the separation of the catalyst, or also by adding a suitable amount of APM to the solution after the separation of the catalyst and dissolving the added APM. And, in the present invention, there can be employed not only an operation step for dissolving APM in the solvent used in the reaction and then starting the reaction, but also an operation for starting the reaction using as the staring material a slurry of APM or a solution dissolving APM as it is, as obtained in a course for production of APM.

Thus obtained reaction solution may be subjected to a crystallizing operation, such as crystallization by cooling, crystallization by concentrating, crystallization by neutralizing, etc., after the concentration as circumstances demand, to crystallize the mixture containing the APM and the APM derivative. In case that there is much APM therein, since it is known that refined (minute) crystals may be formed under stirring, the solution may be subjected to crystallization under standing (static crystallization) or to crystallization under stirring, if required, after the crystallization under standing as described in, for example, Japanese Patent Kokoku Publication JP-B-03-025438.

For a good point (advantage) in the case of the production of the sweetener composition by the process, naturally, the fact that a homogeneously mixed form is achieved as mentioned above, can be given in comparison to that obtained by the process for mixing the crystals of the APM with the crystals of the APM derivative, each separately produced.

As for the sherbet or the slurry obtained in the crystallization, the end product can be obtained by subjecting the same to separation of a solid material from a liquid, drying the solid material, and further granulating (pelletizing), if required.

For the method for separation of a solid material from a liquid, a filtration and a centrifugation are exemplified, and for the method for dryness thereof methods with a vacuum dryer, a fluidized-bed dryer, a spray dryer, a micron dryer and the like are exemplified, and for the method for granulation thereof, dry granulating, wet granulating and the like are exemplified, and however there is no limitation to these exemplified methods.

There is no difficulty, when the sweetener composition obtained in the present invention may be used for a sweetener or a production thereof. For example, it can be easily conducted by applying known methods for production of sweeteners or for use thereof to such case.

### PREFERRED EMBODIMENTS

The present invention is further illustrated specifically by referring to the following examples.

### [EXAMPLE 1]

APM (aspartame; 55.0 g, 0.180 mol) in the water content of 3.9 % by weight and 3,3-dimethylbutylaldehyde (2 ml, 0.016 mol) were added to water (1000 ml) and the resulting solution was maintained at 23 °C. 5% palladium-carbon (5.0 g) was added thereto, and the solution was subjected to a reductive reaction for 2 hours under a flow of hydrogen (H₂) at a flow rate of 100 ml per 1 hour. After that, the flowing of hydrogen was terminated, and the solution was heated to 69 °C to dissolve completely the undissolved crystals. The catalyst was removed by filtration with a filter paper of 0.5 micrometer(µ m), and the resulting filtrate was allowed to stand for static crystallization at 5 °C for 4 hours. Thus obtained a pseudo-solid phase (sherbet) was maintained at 5 °C under stirring overnight to prepare a slurry. Thus obtained slurry was filtrated through a filter paper of 5 µm to separate a solid phase from a liquid phase. The solid phase was washed with 250 ml of water. Thus washed solid phase was dried under reduced pressure at 50°C overnight to obtain 43.6 g of the dried crystals in the water content of 2.7 % by weight ( APM: 95.8 weight %; N-(3,3-dimethylbutyl)-APM: 2.5 weight % in the determination of the high performance liquid chromatography). In the analysis thereof by the thin layer chromatography (TLC), N-[N,N-di(3,3-dimethylbutyl)-L- α -aspartyl]-L-phenylalanine methyl ester which is a dialkylated product was not contained therein. Thus obtained composition was homogeneous (uniform) and was improved in quality of sweetness giving an extremely excellent taste as a sweetener.

### EFFECTS OF INVENTION

In the reaction for reductive alkylation in the present invention, a production of the above-identified by-product is extremely reduced or restrained by using the APM in an amount at least equimolar to the 3,3-dimethylbutylaldehyde.

Further, according to the process in the present invention, the sweetener composition containing the APM and the APM derivative improved in tasting properties can be easily, uniformly and also efficiently produced at a high yield and a high purity by comparison to the process for producing crystals of APM and crystals of APM derivative, each separately, and thereafter mixing the both two types of crystals thus obtained separately.

## Claims

1. In a process for production of N-[N-(3,3-dimethylbutyl)-L-α-aspartyl]-L-phenylalanine methyl ester comprising an reaction of APM with 3,3-dimethylbutylaldehyde for reductive alkylation, the process for production of sweetener composition containing APM and said APM derivative, wherein said APM derivative is produced from a portion of APM by using said 3,3-dimethylbutylaldehyde in an amount at most equimolar to APM in said reaction, the reducing agent is separated from the reaction solution, and said solution is subjected to crystallization followed by the separation of the crystals thus precipitated.

2. A sweetener composition containing the APM and the APM derivative obtained in the process as defined in claim 1.

3. The composition as defined in claim 2, which is a dried sweetener composition.

4. A sweetener comprising the composition as defined in claim 3 and a carrier for sweeteners.

5. The composition as defined in claim 2, wherein said APM and said APM derivative are contained in a ratio of APM to APM derivative being 100 to 0.05 - 50 by weight.

6. The process as defined in claim 1, wherein said 3,3-dimethylbutylaldehyde is used in in amount of 0.0003 - 0.28 mole per 1 mole of APM in said reaction for reductive alkylation.
